# EUROPÄISCHE PATENTANMELDUNG

(11) **EP 1 902 790 A2**
(43) Veröffentlichungstag der Anmeldung: **26.03.2008**
(21) Anmeldenummer: 06026459.5
(22) Anmeldetag: 20.12.2006
(51) Int. Cl.: B09B 3/00, B02C 17/00, F26B 11/04, B01J 19/28, A61L 11/00

(54) **Vorrichtung zur Aufbereitung von Biomasse**

(30) Priorität: 22.09.2006 DE 102006045320
(71) Anmelder: Firma SBM Maschinen GmbH, 87657 Görisried (DE)
(72) Erfinder:
(74) Vertreter: Pfister, Stefan Helmut Ulrich

(57) **Zusammenfassung**

Die Erfindung betrifft eine Vorrichtung zur Aufbereitung von Biomasse, insbesondere von Speiseresten. Sie besteht aus einer in einem Gestell (12) zumindest abschnittsweise um eine Achse (11) drehbar gelagerten Trommel (1) mit antreibbaren Zerkleinerungswerkzeugen (2).

Die Erfindung zeichnet sich dadurch aus, daß eine Trocknungsvorrichtung (3) an der Trommel (1) beziehungsweise am Umfang der Trommel vorgesehen ist und die Trocknungsvorrichtung Trocknungsenergie in die Trommel leitet.

## Beschreibung

Die Erfindung betrifft eine Vorrichtung zur Aufbereitung von Biomasse, insbesondere von Speiseresten, bestehend aus einer in einem Gestell zumindest abschnittsweise um eine Achse drehbar gelagerten Trommel mit antreibbaren zerkleinerungswerkzeugen.

Derartige Vorrichtungen dienen zur Aufbereitung von Biomasse. Als Biomasse sind dabei zum Beispiel Speisereste aus Großküchen, Pizzerien, Gaststätten oder dergleichen zu verstehen. Unter Aufbereitung dieser Biomasse wird verstanden, daß diese für eine Weiterverarbeitung beziehungsweise Entsorgung entsprechend aufzubereiten sind. Das bedeutet, daß die Speisereste zerkleinert werden müssen, zuvor gegebenenfalls vorhandene Fremdkörper wie Bestecke aus Metall oder Kunststoff zu separieren sind und die Speisereste dann in zerkleinerter und gegebenenfalls getrockneter Form einer weiteren Verarbeitung oder einer Deponierung zugeführt werden müssen.

Im Stand der Technik ist es bekannt, die Speisereste dazu zunächst mit Wasser zu verdünnen und gleichzeitig zu zerkleinern, um einen Abtransport aus den Zerkleinerungsvorrichtungen zu ermöglichen. Die so zerkleinerten Speisereste werden dann einer Trocknungsvorrichtung zugeführt. Dazu sind beispielsweise zentrifugenartige Trocknungsvorrichtungen bekannt, mittels derer die Feuchtigkeit aus den Speiseresten herausgeschleudert wird. Die Problematik im Stand der Technik ist zum einen, daß die Kontaminierungen der Flüssigkeit beziehungsweise des Wassers mit den inhaltsstoffen aus den Speiseresten nur schwer kontrollierbar sind. Insbesondere wird dabei auch die Möglichkeit gegeben, die Abwässer mit anderen Kontaminationen zu mischen, was schwer zu kontrollieren ist. Im Weiteren wird natürlich ein recht hoher anlagentechnischer Aufwand betrieben, um die Biomasse entsprechend aufzubereiten, das heißt, zu zerkleinern und zu trocknen.

Ein weiteres Problem, welches bei der zuvor beschriebenen Art der Aufbereitung der Biomasse entsteht, ist, daß nur schwer bestimmte Fremdkörper aus der Biomasse entfernt werden können. Dies kann beispielsweise bei Metallbestecken dazu führen, daß vor allem die Zerkleinerungsvorrichtungen und die Trocknungsvorrichtungen beschädigt beziehungsweise zerstört werden. Für kleinere Einrichtungen, wie beispielsweise Pizzerien oder andere Imbissversorgungseinrichtungen lohnt sich der Aufwand für die Anschaffung einer derartigen Anlage nicht. Es entsteht dadurch allerdings ein recht hoher Aufwand in Form des Abtransportes der Abfälle zu den entsprechenden Entsorgungseinrichtungen.

Es ist eine Vorrichtung zur Zerkleinerung von Gut, insbesondere Fleisch, mit einem drehbar gelagerten, angetriebenen Gefäß bekannt, in dem wiederum eine drehbar gelagerte und angetriebene Messerwelle eingebaut ist. Auf der Messerwelle sind kreisförmig ausgebildete Messer angeordnet.

Ferner ist eine Vorrichtung zum Mischen und Auflockern, Zerkleinern und Umhüllen von pulvrigen und stückigen Stoffen mit einem sich um eine waagerechte Achse drehenden Mischbehälter und einem exzentrisch mit hoher Geschwindigkeit angetriebenen Rotor bekannt.

Es ist auch eine Behandlungsvorrichtung zur Behandlung von organischen Abfällen bekannt. Die Behandlungsvorrichtung ist sowohl als ein biologisch abbauendes Abfallbehandlungssystem als auch als Trocknungssystem einsetzbar.

Aufgabe der Erfindung ist es daher, eine Lösung vorzuschlagen, die mit relativ geringem Aufwand eine Aufbereitung von Biomasse ermöglicht, die insbesondere ohne Zuführung zusätzlichen Wassers auskommt und die im Übrigen die aus dem Stand der Technik bekannten, negativen Auswirkungen bezüglich des Aufbereitens und der Entsorgung von Biomasse beseitigt.

Die Erfindung geht von dem zuvor beschriebenen Stand der Technik aus und schlägt eine Vorrichtung zur Aufbereitung von Biomasse, insbesondere von Speiseresten, bestehend aus einer in einem Gestell zumindest abschnittsweise um eine Achse drehbar gelagerten Trommel mit antreibbaren zerkleinerungswerkzeugen vor, die sich dadurch auszeichnet, daß eine Trocknungsvorrichtung an der Trommel beziehungsweise am Umfang der Trommel vorgesehen ist und die Trocknungsvorrichtung Trocknungsenergie in die Trommel leitet.

Die Trocknungsvorrichtung wird dazu bekanntermaßen, angeordnet und die entstehenden Dämpfe beziehungsweise Gase in die Atmosphäre abgeführt oder über Absaugvorrichtungen zielgerichtet abgeführt. Die Trocknungsvorrichtung nach der Erfindung hat den Vorteil, die Biomasse im Volumen und im Gewicht zum späteren Transport der getrockneten Speisereste deutlich zu reduzieren, aber dennoch so, daß der Brennwert der Biomasse erhalten bleibt. Eventuell entstehende Mikororganismen werden von der Trocknungsenergie zerstört, so daß auch üble Gerüche überhaupt nicht oder nur deutlich reduziert entstehen können. Dennoch bleiben bestimmte Bakterien erhalten, die allerdings erst aktiv werden, wenn wieder Feuchtigkeit in einer zentralen verwertungsanlage zugesetzt wird. Die Trocknungsvorrichtung führt zu einer steuerbaren, schnellen Gewichts- und Volumenreduktion. Diese Vorteile beseitigen die aus dem Stand der Technik bekannten Nachteile. So wird bei Vorrichtungen ohne eine Trocknungsvorrichtung beziehungsweise ohne ein Heizelement das Volumen und das Gewicht des Abfalls nicht reduziert sondern der Abfall lediglich zerkleinert.

Bei einer bekannten Vorrichtung zur Zerkleinerung von Gut sind die Abmessungen der Messer nur geringfügig kleiner als der Durchmesser des Gefässes. Dadurch, daß die Messer geringfügig kleiner als der Durchmesser des Gefässes sind, werden auch metallische Bestandteile, wie beispielsweise Besteckteile, die in den Speisenresten vorhanden sind, dann in der Zerkleinerungsvorrichtung zerstört . Durch den geringen Abstand zum Gefäßdurchmesser kann dies zur Zerstörung des Messers, des Gefässes oder aber sogar zu Blockaden des Antriebs führen. Eine Trocknung erfolgt bei dieser Lösung vor einem Verpacken, beispielsweise im Behälter für einen Abtransport, nicht. Damit sind alle Bestandteile, die in der Biomasse enthalten sind, auch in dem zerkleinerten Gut noch enthalten. Die Erfindung behebt dieses Problem in hervorragender Weise dadurch, daß die Zerkleinerungswerkzeuge, zumindest Abschnittsweise, um eine Achse drehbar gelagert sind und die im Stand der Technik bekannten Probleme dadurch nicht auftreten.

Behandlungsvorrichtungen mit einer Trocknungsvorrichtung weisen den Nachteil auf, daß die Verarbeitungszeit lang dauern kann, insbesondere wenn Mikroorganismen eingesetzt werden. Für das biologisch verarbeitende System wird der rohe Abfall mit biologischen Chips, Mikroorganismen gemischt. Damit die Mikroorganismen hoch aktiv sind, wird die Behandlungstemperatur auf ungefähr circa 60°C gehalten. Beim Trocknungssystem wird der rohe Abfall ohne biologische Mikroorganismen im Behandlungsbehälter angeordnet. Eine Zerkleinerungsvorrichtung weist eine senkrecht stehende Rotationsachse mit über die Höhe verteilten, rotierenden Messern auf. Durch die vorher beschriebene Arbeitsweise kann ein starker Geruch bei einer Erwärmungstemperatur, von wie beispielsweise vorgeschlagen 60°C auftreten. Der Stand der Technik setzt zur Geruchsreduktion biologische Mikroorganismen ein. Der mit einer Trocknungsvorrichtung verarbeitete und behandelte Abfall dient im Endprodukt als Düngemittel und nicht als Biomasse mit einem verdichteten und erhöhten Brennwert. Ein gewichtiger Nachteil des Standes der Technik ist, daß durch die Zerkleinerungsvorrichtung Metall oder Plastik mit eingeführt werden kann, das die Zerkleinerungsvorrichtung beschädigt oder zerstört.

Die Erfindung bringt auch den Vorteil, daß die getrocknete Restbiomasse biologisch inaktiv und somit geruchsneutral nach der Aufbereitung ist und sich sehr gut für ein Logistikkonzept eignet, nachdem diese getrocknete Biomasse mit einem noch vorhandenen Brennwert eingesammelt wird und in einer thermischen Energiegewinnungsanlage zur Verwertung gebracht wird.

Bei einer bevorzugten Ausführungsform ist die Achse der Trommel horizontal beziehungsweise im Wesentlichen horizontal ausgerichtet. Dies hat den Vorteil, daß die antreibbaren Zerkleinerungswerkzeuge effizient die gesamte Biomasse und nicht einseitig zerkleinern, zusammen mit einem gleichzeitig stattfindenden Trocknungsvorgang mit der Trocknungsvorrichtung.

Besonders effizient erfolgt die Trocknung, wenn die Trocknungsvorrichtung zum Beispiel als IR (infrarot)-Lampe, Wärmeluftgebläse oder wärmestrahler ausbildbar ist.

Die Trommel ist selbstverständlich entsprechend einer günstigen Ausführungsform der Erfindung mit kleinen Öffnungen ähnlich einer Waschmaschinentrommel,versehen, über die gegebenenfalls Feuchtigkeit entweichen kann.

Die Trocknungsvorrichtung ist entsprechend einer Variante der Erfindung als Wärmestrahler oder als Warmluftgebläse vorgesehen. Dieses Warmluftgebläse wird bevorzugt unterhalb oder seitlich der Trommel angeordnet und ihr gegenüberliegend ist dann eine Abluftvorrichtung oder Abluftabsaugvorrichtung vorgesehen, über die die mit Feuchtigkeit angereicherte Warmluft dann abgeführt werden kann. Der Abluftvorrichtung nachgeordnet können Kondenswassereinrichtungen ober dergleichen vorgesehen sein, über die die Feuchtigkeit aus der Luft wieder abgeschieden wird. Allgemein hat die Trocknungsvorrichtung die Aufgabe, Trocknungsenergie (zum Beispiel wärme oder auch andere Strahlung) in die Trommel zu leiten.

Eine besonders günstige Variante der Erfindung schlägt vor, als Trocknungsvorrichtung ein Mikrowellen erzeugendes Gerät vorzusehen. Dieses Mikrowellen erzeugende Gerät erzeugt ein Mikrowellenfeld, welches auf das aufzubereitende Gut gerichtet wird und die dieses Gut erwärmt. Durch die Erwärmung kann die Feuchtigkeit aus dem Gut entweichen. Gleichzeitig oder auch zeitlich versetzt wird natürlich das Zerkleinerungswerkzeug durch das periodische Hineinschlagen in das aufzubereitende Gut für eine weitere Zerkleinerung und gleichzeitig eine Umwälzung sorgen. Das aufzubereitende Gut wird demnach vollständig zerkleinert und gleichzeitig so weit getrocknet, daß es nur noch mit geringer Restfeuchte krümel- oder pelletförmig oder als nahezu staubförmige Masse nach dem Trocknungsprozeß aus der Trommel entnommen werden kann.

Durch das völlig neue erfindungsgemäße Konzept, gegenüber den aus dem Stand der Technik bekannten Lösungen, wird jetzt eine Zerkleinerung der Biomasse, eine Trocknung der Biomasse sowie ein autarker Betrieb bezüglich Abwasser und Wasserzulauf erreicht. Anlagentechnisch spart man gegenüber dem bisher bekannten Betrieb der Zerkleinerung in einer zerkleinerungsvorrichtung mittels Wasserzuführung, dem Abpumpen der so zubereiteten Biomasse in eine Trocknungsvorrichtung in Form einer Zentrifuge, die Zentrifuge und zumindest die Pumpe ein. Für die Zerkleinerungsvorrichung hat man jetzt die neuartige Vorrichtung zur Aufbereitung von Biomasse vorgesehen, in der sowohl eine Zerkleinerung als auch gleichzeitig eine Trocknung erfolgt. Nach dem Trocknen wird die aufbereitete Biomasse selbstverständlich in einer Entleerungsstellung entleert. Die aufbereitete Biomasse kann dann von den Fremdstoffen, wie zum Beispiel Plastik, Metall und so weiter in Form üblicher Trennverfahren getrennt werden. Dazu dienen beispielsweise Trennverfahren wie Windsichtung für Fremdpartikel aus Kunststoff oder für das Metall Trennung in Form von Magneten oder durch Metalldetektoren.

Die Trocknungsvorrichtung ist, wie bereits erwähnt; durch ein Mikrowellen erzeugendes Gerät angegeben, welches ein Mikrowellenfeld erzeugt. Dieses Mikrowellenfeld wird zumindest von einer Strahlungsquelle auf das aufzubereitende Gut gerichtet. Selbstverständlich ist es erfindungsgemäß auch vorgesehen, daß mehrere Strahlungsquellen das Mikrowellenfeld erzeugen und damit für eine optimale Erwärmung und damit verbundene Trocknung des aufzubereitenden Gutes führen.

Bevorzugt ist es dabei, wenn die Strahlungsquelle die Mikrowellen durch eine oder mehrere Glasluken, die am Umfang der Trommel angeordnet sind, auf das aufzubereitende Gut beziehungsweise die Biomasse sendet.

Ein weiterer Aspekt der erfindungsgemäßen Lösung ist dadurch angegeben, daß zusätzlich oder alternativ wenigstens eine Strahlungsquelle für UV-Strahlen als Trocknungsvorrichtung und/oder als Keimdestruktionsvorrichtung, insbesondere von Mikroorganismen, vorgesehen ist. Man erreicht neben dem bereits beschriebenen Effekt der, insbesondere schnellen, Trocknung gleichzeitig noch eine Befreiung von Keimen, wodurch die Biomasse nicht so schnell verdirbt und einer späteren Weiterverarbeitung problemlos und ohne Zeitdruck zuführbar ist.

Die Erfindung schlägt in einer bevorzugten Weiterbildung vor, daß das Mikrowellengerät derart ausgebildet ist, daß es die Wellen getaktet aussendet. Dies hat den Vorteil, daß aufgrund der ständigen Beaufschlagung mit Mikrowellen sich das aufzubereitende Gut nicht entzünden kann. Vielmehr wird durch die Taktung erreicht, daß in einem ersten Takt die Biomasse erwärmt wird und im nächsten Takt, in dem beispielweise keine Mikrowellen ausgesendet werden, sich die Biomasse wieder abkühlt und gleichzeitig allerdings entsprechende Feuchtigkeit entweichen kann. Der gleiche Effekt wird natürlich auch bei einer getakteten Abgabe der Trocknungsenergie durch die Trocknungsvorrichtung erreicht. Eine punktuelle Überhitzung mit der Gefahr einer Entzündung des Trocknungsgutes wird vermieden, eine gleichmäßige Trocknung wird erreicht.

Dazu ist es erfindungsgemäß in einer weiteren Variante vorgesehen, eine Steuerung zumindest für die Taktung des Mikrowellengerätes vorzusehen. Selbstverständlich kann die Steuerung neben der Taktung für das Mikrowellengerät gleichzeitig auch eine Taktung für die UV-Strahlungwelle vorsehen. Die Taktung kann dabei derart erfolgen, daß zunächst Mikrowellen auf die Biomasse einwirken und anschließend, nach dem das Mikrowellengerät abgeschaltet ist, die UV-Strahlen auf die Biomasse gesendet werden. Selbstverständlich können auch beide Wellen gleichzeitig auf die Biomasse einwirken. Dies ist eine Aufgabe, die jede Steuerung heutzutage ohne weiteres realisieren kann.

Die Trommel der Vorrichtung ist so positioniert, daß keine Strahlung, egal ob mit Mikrowellen oder UV-Strahlen gearbeitet wird, austreten kann.

Die Steuerung kann selbstverständlich auch die Bewegungsabläufe der Trommel und/oder des oder der Zerkleinerungswerkzeuge realisieren. Die Erfindung ist hier keineswegs eingeschränkt.

Die Erfindung schlägt auch vor, daß eine Abluft- beziehungsweise Absaugvorrichtung zum Absaugen der beim Trocknen austretenden Wasserdämpfe und Gase vorgesehen ist.

Die erfindungsgemäße Vorrichtung zeichnet sich entsprechend einer Weiterbildung auch dadurch aus, daß eine bevorzugt verschließbare Öffnung zum Füllen und/oder Entleeren der Trommel vorgesehen ist. Dazu ist es erfindungsgemäß auch vorgesehen, daß die Öffnung der Trommel wenigstens an einer Befüllstellung, an einer Bearbeitungsstellung und an einer Entleerungsstellung durch Drehen beziehungsweise Bewegen der Trommel positionierbar ist. In der ersten Stellung wird beispielsweise die Biomasse chargenweise in die Trommel eingefüllt. Danach wird die Öffnung verschlossen und die Trommel in eine Bearbeitungsstellung bewegt. In dieser Bearbeitungsstellung wirken die Zerkleinerungswerkzeuge auf die Biomasse ein. Gleichzeitig wird die Biomasse durch Beaufschlagung mit Trocknungsenergie wie Mikro- oder UV-Strahlen getrocknet.

Die Bearbeitungsstellung ist dabei bevorzugt derart vorgesehen, daß die Absaugvorrichtung sich in dieser über der Öffnung befindet. Sowohl die Öffnung aber auch die Trommel kann an ihrem Umfang kleinere Öffnungen aufweisen, über die Feuchtigkeit zusätzlich oder alternativ auszutreten vermag. Selbstverständlich kann die Trommel auch siebartig ausgebildet sein. Die Trommel kann entsprechend einer Weiterbildung auch insgesamt geschlossen sein. Nur in der Öffnung sind dann weitere kleinere Öffnungen vorgesehen, über die die Feuchtigkeit austreten kann. Hier sind verschiedene Varianten von der Erfindung umfaßt.

Die Entleerungsstellung ist bevorzugt derart vorgesehen, daß die getrocknete Biomasse senkrecht nach unten in einen dort positionierten Auffangbehälter gelangen kann. Die Trommel wird dazu in die Entleerungsstellung gedreht und die Öffnung geöffnet. Der Auffangbehälter kann dabei unterschiedlich ausgeführt sein. Es sind beispielsweise auch Kleincontainer möglich, die problemlos transportiert werden können. Auch ein Ausblasen mittels Druckluft, oder ein mechanisches Ausrütteln des aufgefangenen Gutes aus dem Auffangbehälter schlägt die Erfindung entsprechend einer weiteren Variante vor. Das ausgeblasene Gut kann dann beispielsweise in einem Silo aufgefangen werden, von dem aus eine weitere Verteilung erfolgt oder aber das Silo insgesamt abtransportiert werden kann, wenn größere Mengen an Biomasse anfallen.

In den Auffangbehälter ist, entsprechend einer Fortbildung der erfindungsgemäßen Lösung, ein Transportbehältnis einlegbar, das für einen Weitertransport der aufbereiteten Masse geeignet ist.

Es ist selbstverständlich, daß auch als Zerkleinerungswerkzeug alle aus dem Stand der Technik bekannten Zerkleinerungswerkzeuge dienen können.

Vorteilhafterweise ist als Zerkleinerungswerkzeug in der Trommel wenigstens ein relativ zur Antriebsachse rotierbarer und verschiebbarer Hammer vorgesehen. Bevorzugt ist eine Vielzahl von Hämmern vorgesehen.

Durch die Relativverschiebung des Zerkleinerungswerkzeuges bezüglich der Trommel ist es jetzt gegeben, daß die in die Trommel eingefüllte Biomasse aufgrund einer Drehbewegung des Werkzeuges und insbesondere aufgrund der Relativbewegung der Hämmer bezüglich der Trommel sozusagen durch periodisches Hineinschlagen in das aufzubereitende Gut dieses Zerkleinern, Wenden und Mischen. Dies hat beispielsweise den Vorteil, daß kein zusätzliches Wasser hinzugegeben werden muß und Fremdstoffe, wie zum Beispiel Plastik, Metall den Betrieb nicht stören und in der Regel unzerstört hinterher wieder herausgefördert werden. Die Vorrichtung hat den Vorteil, daß sie relativ langsam läuft, wobei insbesondere die Relativbewegung des Zerkleinerungswerkzeuges dazu führt, daß die Biomasse sehr fein zerkleinert wird und gleichzeitig in der Biomasse befindliche Fremdkörper nicht aufgrund der Bewegung des Hammers zerstört beziehungsweise deformiert werden oder aber zur Beschädigung der Trommel oder des Werkzeuges führen. Die Fremdkörper werden gewissermaßen mit dem Hammer oder den Hämmern mitbewegt beziehungsweise können an den Hämmern vorbeigleiten, ohne diese zu zerstören.

Die Achse der Trommel ist entsprechend einer bevorzugten variante der Erfindung horizontal beziehungsweise im Wesentlichen horizontal ausgerichtet. Die Trommel hat weiterhin wenigstens eine Öffnung in ihrer Mantelfläche. Diese Öffnung dient zum Befüllen der Trommel. Sie dient natürlich auch zum Entleeren der zerkleinerten Biomasse. Das erfindungsgemäße Konzept unterscheidet sich insbesondere von den aus dem Stand der Technik bekannten Lösungen dadurch, daß die Trommel nicht mehr in Form einer äußerst schnellen Drehbewegung, wie dies bei Zentrifugen der Fall ist, sich bewegt, sondern sich bevorzugt lediglich nur abschnittsweise, nämlich zum Befüllen, in eine Bearbeitungsstellung und in eine Entleerungsstellung bewegt. Dadurch können das sich im Inneren der Trommel befindliche Hammerwerk beziehungsweise die Zerkleinerungswerkzeuge die Zerkleinerungsaufgabe vollständig übernehmen.

Die Zerkleinerungswerkzeuge sind bevorzugt in Art von Hämmern ausgebildet, wobei der oder die Hämmer für ein periodisches Hineinschlagen in das aufzubereitende Gut ansteuerbar sind.

Bevorzugt sind die Zerkleinerungswerkzeuge auf die gesamte Biomasse in der Trommel während der Aufbereitung einwirkend angeordnet. Die gesamte Biomasse ist somit während der Aufbereitung von dem Zerkleinerungswerkzeug vollständig erfaßbar und zerkleinerbar. Somit wird ein hoher Zerkleinerungsgrad erzielt.

Der oder die Hämmer sind auf der Welle drehbar gelagert, derart, daß durch eine Drehbewegung der Welle der Hammer in der Aufwärtsbewegung nach oben transportiert wird und anschließend aufgrund der Schwerkraft schneller in der Trommel sich bewegt als dies durch die Drehbewegung der welle verursacht wird. Dazu ist beispielsweise ein Mitnehmer vorgesehen, der den Hammer bei einer Drehbewegung der Welle, insbesondere beim Aufwärtsbewegen des Hammers mitnimmt.

Der Drehpunkt beziehungsweise die Achse für die Drehbewegung des Hammers ist dabei nicht identisch mit der Achse der Welle der Trommel, sondern ist außermittig dieser Achse vorgesehen.

Auch der Mitnehmer auf der Welle, der beispielsweise sich auf einer auf der Welle befindlichen Hülse befinden kann, ist beabstandet, also außerhalb vom Drehpunkt für den Hammer angeordnet, um den zuvor beschriebenen Ablauf bewirken zu können. Selbstverständlich kann auch die Hülse derart ausgebildet sein, daß eine Mitnahme des Hammers durch bestimmte Ausklinkungen erfolgen kann. Dazu kann beispielsweise entsprechend einer Variante der Erfindung der Hammer auch an der Hülse drehbar gelagert sein, die dann den Hammer nach oben mitnimmt, wenn die Welle sich in Aufwärtsbewegung befindet.

Bevorzugt ist es, wenn eine Vielzahl von Hämmern auf der Welle vorgesehen ist, wobei die Hämmer beabstandet voneinander und bezüglich ihres Drehpunktes versetzt zueinander angeordnet sind. Dadurch wird eine hervorragende Zerkleinerung und gleichzeitige Vermischung des aufzubereitenden Biomaterials bewirkt.

Vorteilhafterweise ist es entsprechend der Erfindung auch vorgesehen, für jeden Hammer einen Mitnehmer anzuordnen. Dies kann entweder durch Anordnung von geteilten Hülsen auf der Welle geschehen, die in unmittelbarer Nähe des Hammers angeordnet werden. Selbstverständlich kann man auch eine Hülse auf der Welle vorsehen, die entsprechende Ausnehmungen für die entlang der Welle vorgesehenen Hämmer besitzt.

Bevorzugt ist es dabei weiterhin, wenn die Ansteuerung des Hammers durch die Steuerung der vorrichtung und/oder der Trommel realisiert wird. Die Hämmer müssen nicht zwingend als Hämmer in Form von Kugelhämmern oder üblicherweise ausgebildeten Hämmern ausgebildet sein. Die Hämmer sind beispielsweise auch lediglich stabförmig ausgebildet.

Bevorzugt sind die Hämmer derart aufgehängt, daß jeweils das vordere Ende eines Hammerkopfes auf das hintere Ende eines Hammerkopfes eines vorhergehenden Hammers aufschlägt. Somit wird die Biomasse in der zerkleinerungsvorrichtung in kleine Stückchen beziehungsweise Teile zerquetscht.

Eine vorteilhafte Weiterbildung der Erfindung schlägt vor, daß die Hämmer flügelartig ausgebildet und in Art eines Flügelrades auf einer Hülse drehbar um die Achse der Trommel und in Richtung der Achse relativ zur Tommel verschiebbar angeordnet sind.

Mit diesem Konzept der Erfindung läßt sich eine sehr günstige Zerkleinerung des zu zerkleinernden Gutes erreichen, ohne daß die Trommel in eine ständige Drehbewegung versetzt werden muß. Insbesondere ist es nicht erforderlich die Trommel mit hohen Drehzahlen laufen zu lassen.

Die Erfindung schlägt dabei vor, daß auf jeder Seite der Trommel wenigstens ein Flügelrad vorgesehen ist, welche sich wechselweise in das aufzubereitende Gut hinein bewegen beziehungsweise aus diesem heraus bewegen. Mit der Relativbewegung ist dabei eine Bewegung des Flügelrades entlang der Achse in Richtung auf die Trommelmitte und zurück gemeint.

Die Flügelräder oder aber der oder die Hämmer sind dabei auf bevorzugt teleskopisch ausgebildeten Hülsen angeordnet, die aufgrund ihrer teleskopischen Ausbildung die Relativbewegung bezüglich der Trommel bewirken können. Die Ansteuerung kann dabei hydraulisch, elektromechanisch oder mechanisch erfolgen. Selbstverständlich können diese Hülsen auch mit Zug- oder Druckfedern ausgestattet sein, die zumindestens eine Bewegungsrichtung der Relativbewegung des Hammers oder des Flügelrades bewirken beziehungsweise abdecken.

Die Steuerung regelt selbstverständlich auch zusätzlich oder alternativ in einer weiteren Variante der Erfindung die Drehbewegung der Trommel, das Befüllung und Entleeren sowie das Absaugen der Wasserdämpfe beziehungsweise Gase. Um eine optimale Trocknung der Biomasse zu gewährleisten ist es erfindungsgemäß ebenfalls entsprechend einer weiteren Variante der Erfindung vorgesehen eine Feuchtigkeitsanzeige und/oder Feuchtigkeitsregelung für das aufzubereitende Gut vorzusehen. Die Feuchtigkeitsregelung ist dabei so ausgebildet, daß sie die Trocknungsvorrichtung bei vorgegebenen Werten ein- beziehungsweise ausschaltet.

Das Gestell der Trocknungsvorrichtung ist bevorzugt rahmenartig ausgebildet. Die Trommel ist dabei in dem Gestell insbesondere drehbar gelagert. Besonders bevorzugt ist es, wenn die Trommel in dem Gestell federgelagert ist, zum Beispiel um die "Schläge" der Hämmer abzufangen.

Die Erfindung ist sehr variabel einsetzbar. Sie ist zum einen dafür vorgesehen, verhältnismäßig geringe Mengen an Biomasse aufzubereiten, in dem das Gewicht und das Volumen durch einen thermischen Wasserentzug reduziert wird. Sie eignet sich dafür zum Beispiel auch für Kleingaststätten und so weiter. Der Trocknungs- bzw. Aufbereitungsvorgang dabei ist zeitlich beliebig einstellbar, üblicherweise wird mit einer langen Bearbeitungszeit vorgegangen, was keine übermäßige Überhitzung (Verkohlung) des Gutes bedeutet und auch die Anlage insgesamt bezüglich der mechanischen Ausgestaltung einfacher werden läßt. Das Gut wird nur soweit erhitzt, daß möglichst umfassend das Wasser aus dem Gut entweicht, der Brennwert des Gutes zur späteren thermischen Energieerzeugung aber erhalten bleibt. Mit der erfindungsgemäßen Vorrichtung wird eine erhebliche Gewichtsreduktion der eingebrachten Biomasse um mindestens 75 bis 80 % erreicht, durch den Einsatz der Steuerung erfolgt die Aufbereitung ab Einfüllen der Biomasse bis zum Entleeren (gegebenenfalls auch mit dem Entleeren) automatisch.

Die Erfindung wird nachfolgend anhand von Ausführungsbeispielen weiter beschrieben. Es zeigen:
- Fig. 1: eine schematische Darstellung einer Ausführungsform der Erfindung und
- Fig. 2: eine weitere Ausgestaltung der erfindungsgemäßen Vorrichtung mit Trocknungseinrichtung.

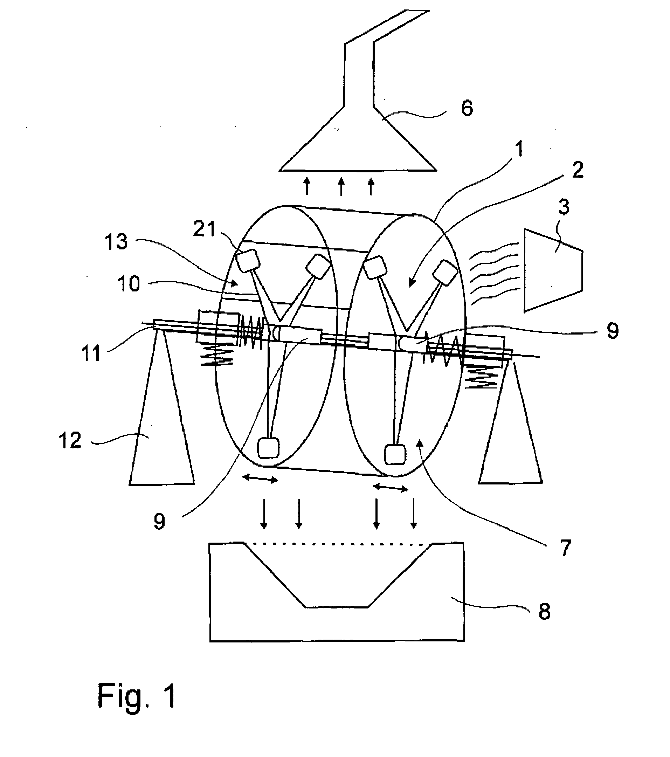

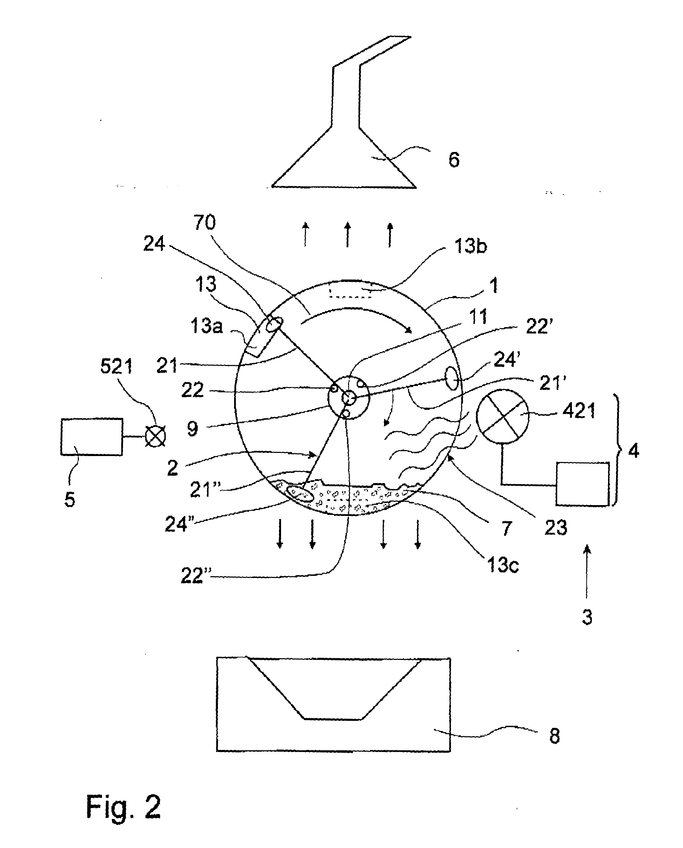

Die Figur 1 zeigt in einer schematischen Darstellung zumindest ausschnittsweise Teile der Vorrichtung nach der Erfindung. Mit dem Bezugszeichen 1 ist die Trommel bezeichnet. In der Trommel 1 befinden sich zwei Flügelräder 10 als Zerkleinerungswerkzeug 2, das in einer Seite nur schematisch angedeutet als Hammer 21 ausgebildete Flügelräder 10 besitzt. Die Flügelräder 10 können dabei entlang einer Achse 11 in Richtung auf die Mitte der Trommel 1 und zurück bewegt werden. Dies wird schematisch durch die Pfeile unterhalb der Trommel 1 angedeutet. Die Trommel 1 ist auf der Achse 11 angeordnet, wobei die Trommel 1, wie lediglich angedeutet, federgelagert in dem Gestell 12 sich befindet. Unterhalb der gesamten Vorrichtung befindet sich ein Auffangbehälter 8, in den das aufzubereitende Gut 7 nach der Aufbereitung aus der Trommel 1 entleert wird. Über der Trommel 1 befindet sich eine Abluft- beziehungsweise Absaugvorrichtung 6. Das aufzubereitende Gut 7 befindet sich in der Trommel 1. Am Außenumfang der Trommel 1 befindet sich eine Öffnung 13, um das Gut 7 aufzunehmen und nach einer Aufbereitung in den Auffangbehälter 8 wieder abzugeben. Die Trommel 1 wird dabei lediglich zum Befüllen, zum Bearbeiten und zum Entleeren in jeweilige, in der Fig. 2 gezeigte und angedeutete Stellungen 13a, 13b, 13c verstellt. Durch die Hin- und Herbewegung der Flügelräder 10 beziehungsweise der Hämmer 21 und deren mögliche Drehbewegung um die Achse 11 ist eine Zerkleinerung des Gutes 7 gegeben. Dabei erfolgt eine laufende Zerkleinerung des gesamten Gutes 7 während der rotierenden Aufbereitung in der Trommel 1. Durch die Abluft- beziehungsweise Absaugvorrichtung 6 wird bewirkt, daß austretende Feuchtigkeit beziehungsweise Gase aus der Trommel 1 abgesaugt wird.

Wie in den vorhergehenden Ausführungsbeispielen beziehungsweise varianten beschrieben, wird bevorzugt das auszubereitende Gut 7 durch eine Trocknungsvorrichtung 3 (siehe auch Fig. 2) gleichzeitig getrocknet. Dazu ist es beispielsweise möglich ein Warmluftgebläse oder Wärmestrahler an der Trommel 1 vorzusehen, um das aufzubereitende Gut 7 zu trocknen. Nachdem das aufzubereitende Gut 7 getrocknet ist, wird es, wie ebenfalls nur mit Pfeilen schematisch angedeutet, in den Auffangbehälter 8 entleert. Auf der Achse 11 sind die Flügelräder 10 auf einer Hülse 9 gelagert. Schematisch angedeutet ist hier lediglich eine Feder, die als Druckfeder belastbar ist. Die Druckfeder bewirkt, daß sich bei Lösen einer entsprechenden Sperre das Flügelrad in Richtung auf die Mitte der Trommel 1 bewegt. Die Rückzugsbewegung kann dann elektromotorisch beispielsweise bewirkt werden. Selbstverständlich kann dies auch mechanisch gelöst werden oder aber wie zuvor in weiteren ,Ausführungsvarianten beschrieben durch eine teleskopische Ausbildung in Form von Hydraulikzylindern oder dergleichen.

Die gesamte Vorrichtung arbeitet derart, daß die Trommel 1 mit dem aufzubereitenden Gut 7 gefüllt wird. Danach wird die Trommel 1 bevorzugt in einer in der Fig. 2 angedeuteten Bearbeitungsstellung 13b gedreht. Dort verbleibt sie während der Bearbeitung und die Hämmer 21 beziehungsweise Flügelräder 10 bewirken durch ein periodisches Hineinschlagen in das aufzubereitende Gut 7 eine Zerkleinerung desselben. Dazu können die Flügelräder 10 selbstverständlich zusätzlich noch in eine Drehbewegung versetzt werden. Durch die Zerkleinerung, insbesondere die Zerkleinerung mit langsam laufenden Zerkleinerungswerkzeugen 2 wird vermieden, daß in dem aufzubereitenden Gut 7 vorhandene Fremdkörper erstens selbst zerstört und zweitens die Vorrichtung beschädigen oder zerstören können. Gemeint sind hierbei Essbestecke oder andere Abfälle, wie sie in Großküchen, Pizzerien, in Küchen von Restaurants oder dergleichen anfallen. Das Gestell 12 ist bevorzugt rahmenartig ausgebildet. Dies ist ebenfalls lediglich schematisch angedeutet. Die Achse 11 ist am Gestell 12 bevorzugt ebenfalls federgelagert. Dies ist ebenfalls lediglich schematisch angedeutet.

Die Figur 2 zeigt in einer schematischen Darstellung eine verbesserte Variante der zuvor beschriebenen Ausführungsform der Erfindung. Die Bezugszeichen, wie sie in der Figur 1 vorgestellt wurden, werden in gleicher Weise wieder verwendet, so daß auf deren erneute Vorstellung zumindest teilweise verzichtet werden kann. Die Figur 2 ist in einer Seitenansicht zu verstehen. Zusätzlich zu den bereits beschriebenen Merkmalen ist in der Trommel 1 die Öffnung 13 schematisch angedeutet.

In der Stellung mit dem Bezugszeichen 13a wird die Befüllstellung angedeutet. Die Stellung 13b bezeichnet die Arbeits- beziehungsweise Bearbeitungsstellung und in der Stellung 13c ist die Entleerungsstellung bezeichnet. Die Trommel 1 wird mit dem aufzubereitenden Gut 7 in der Befüllstellung 13b gefüllt. Danach wird die Trommel 1 in die Bearbeitungsstellung 13a bewegt. Dann werden die Hämmer 21 in eine Drehbewegung 70 (siehe Pfeil) versetzt, so daß ein rythmisches Einschlagen auf das aufzubereitenden Gut 7 bewirkt wird. Die Hämmer 21, 21', 21'' sind in der in der Fig. 2 dargestellten Variante der Erfindung pendelnd beziehungsweise drehbar an der Welle 11 beziehungsweise an der Hülse 9 gelagert. Um ein periodisches Hineinschlagen der Hämmer 21 mit den Hammerköpfen 24 zu ermöglichen, ist in dieser Ausführungsform ein Mitnehmer 22, 22', 22" vorgesehen, der den Hammer 21 bei der Aufwärtsbewegung mitnimmt. Befindet sich der Hammer 21 in der oberen Stellung und überschreitet seinen Schwerpunkt, fällt er aufgrund der Schwerkraft schneller nach unten und zerkleinert dabei das aufzubereitende Gut 7. Dabei schlägt gegebenenfalls das vordere Ende des Hammerkopfs 24' auf das hintere Ende des vorausgehenden Hammerkopfs 24'' und zerquetscht dazwischenliegendes Gut 7. Unten angelangt verharrt er in der unteren Stellung so lange, bis der Mitnehmer 22' aufgrund der langsameren Drehbewegung 70 der Achse 11 an den Hammer 21' beziehungsweise seinen Stiel anstößt und diesen dann wieder nach oben mitnimmt. Entlang der Achse 11 sind selbstverständlich mehrere Hämmer 21 vorgesehen. Dies ist lediglich schematisch angedeutet. Die Hämmer 21 sind dabei beabstandet voneinander und insbesondere bezüglich ihres Drehpunktes versetzt auf der Welle beziehungsweise an der Hülse angeordnet. Dadurch wird das aufzubereitende Gut 7 zerkleinert. Gleichzeitig werden die in dem Gut 7 vorhandenen Feuchtigkeitsbestandteile freigesetzt.

Am Umfang der Trommel 1 ist schematisch angedeutet eine Trocknungsvorrichtung 3, die in der dargestellten Variante ein Mikrowellen erzeugendes Gerät 4 beinhaltet, welches die Mikrowellen durch Strahler 421 auf die Trommel 1 richtet. Die Strahlen gelangen durch zumindest eine Glasluke 23 in das Innere der Trommel 1. Die Glasluke 23 ist natürlich für die verwendete Wellenlänge der Strahlung transparent beziehungsweise nur gering absorbierend. Des weiteren kann eine Strahlungsquelle 5 für UV-Strahlen beidseitig der Vorrichtung vorgesehen sein, die die erzeugten UV-Strahlen über eigene Strahler 521 ebenfalls auf das aufzubereitende Gut 7 in der Trommel 1 richtet. Wasserdampf und andere Gase entweichen in der Arbeitsstellung der Trommel 1 durch die obere Stellung der Öffnung 13 und werden von der Absaugvorrichtung 6 abgesaugt.

Es ist auch möglich, eine zusätzliche Strahlungsquelle 5 für die UV-Strahlen vorzusehen. Durch die Beaufschlagung mit UV-Strahlen wird neben einer Trocknung zusätzlich noch eine Befreiung beziehungsweise Zerstörung und Vernichtung von möglicherweise vorhandenen Keimen und Mikroorganismen erreicht, so daß das aufbereitete Gut 7 nach der Zerkleinerung und der Trocknung keimfrei in den Auffangbehälter 8 gelangen kann.

Die Trocknung des Gutes 7 erfolgt in der Weise, daß die Feuchtigkeit aus dem Gut 7 so weit wie möglich zur Gewichts- und Volumenreduzierung aus dem Gut 7 entzogen werden, so daß lediglich trocknes Gut 7 mit einem Restbrennwert übrig bleibt. Eventuelle Mikroorganismen können dabei auch zerstört und vernichtet werden. Das getrocknete Gut 7 dient als Brennmaterial in thermischen Energiegewinnungsanlagen.

Aus dem Auffangbehälter 8 wird das getrockene Gut 7 entweder durch Ausblasen zu Silos oder größeren Transportbehältern weiter transportiert oder aber dort gesammelt und, nachdem der Auffangbehälter 8 gefüllt ist, dann abtransportiert und durch einen neuen Behälter ersetzt. Nach der Erfindung ist es auch möglich, kleinere Transportbehältnisse in dem Auffangbehälter 8 zu positionieren, die einen einfachen Weitertransport zur Verwertungsanlage ermöglichen. Diese Behältnisse können als Karton oder Sack ausgebildet sein. Bevorzugt sind diese Behältnisse aus verrottbarem Material.

Insbesondere wird auf die zeichnerischen Darstellungen für die Erfindung als wesentlich verwiesen.

Die jetzt mit der Anmeldung und später eingereichten Ansprüche sind Versuche zur Formulierung ohne Präjudiz für die Erzielung weitergehenden Schutzes.

Sollte sich hier bei näherer Prüfung, insbesondere auch des einschlägigen Standes der Technik, ergeben, daß das eine oder andere Merkmal für das Ziel der Erfindung zwar günstig, nicht aber entscheidend wichtig ist, so wird selbstverständlich schon jetzt eine Formulierung angestrebt, die ein solches Merkmal, insbesondere im Hauptanspruch, nicht mehr aufweist.

Die in den abhängigen Ansprüchen angeführten Rückbeziehungen weisen auf die weitere Ausbildung des Gegenstandes des Hauptanspruches durch die Merkmale des jeweiligen Unteranspruches hin. Jedoch sind diese nicht als ein Verzicht auf die Erzielung eines selbständigen, gegenständlichen Schutzes für die Merkmale der rückbezogenen Unteransprüche zu verstehen.

Merkmale, die bislang nur in der Beschreibung offenbart wurden, können im Laufe des Verfahrens als von erfindungswesentlicher Bedeutung, zum Beispiel zur Abgrenzung vom Stand der Technik beansprucht werden.

Merkmale, die nur in der Beschreibung offenbart wurden, oder auch Einzelmerkmale aus Ansprüchen, die eine Mehrzahl von Merkmalen umfassen, können jederzeit zur Abgrenzung vom Stande der Technik in den ersten Anspruch übernommen werden, und zwar auch dann, wenn solche Merkmale im Zusammenhang mit anderen Merkmalen erwähnt wurden beziehungsweise im Zusammenhang mit anderen Merkmalen besonders günstige Ergebnisse erreichen.

## Patentansprüche

1. Vorrichtung zur Aufbereitung von Biomasse, insbesondere von Speiseresten, bestehend aus einer in einem Gestell (12) zumindest abschnittsweise um eine Achse (11) drehbar gelagerten Trommel (1) mit antreibbaren Zerkleinerungswerkzeugen (2), **dadurch gekennzeichnet, daß** eine Trocknungsvorrichtung (3) an der Trommel (1) bzw. am Umfang der Trommel (1) vorgesehen ist und die Trocknungsvorrichtung (3) Trocknungsenergie in die Trommel (1) leitet.

2. Vorrichtung nach Anspruch 1, **dadurch gekennzeichnet, daß** die Achse (11) der Trommel (1) horizontal bzw. im wesentlichen horizontal ausgerichtet ist.

3. Vorrichtung nach einem oder beiden der vorhergehenden Ansprüche, **dadurch gekennzeichnet, dass** die Trocknungsvorrichtung (3) als wärmestrahler, IR-Lampe oder Warmluftgebläse vorgesehen ist und/oder als Trocknungsvorrichtung (3) ein Mikrowellen erzeugendes Gerät (4) vorgesehen ist und/oder die Trocknungsvorrichtung (3) durch ein Mikrowellenfeld angegeben ist, welches in dem Mikrowellengerät (4) erzeugt und von zumindest einer beispielsweise als Strahler (421) ausgebildeten Strahlungsquelle auf das aufzubereitende Gut (7) gerichtet wird.

4. Vorrichtung nach einem oder mehreren der vorhergehenden Ansprüche, **dadurch gekennzeichnet, dass** wenigstens ein Strahler (421) die Mikrowellen auf eine oder mehrere Glasluken (23) richtet, die am Umfang der Trommel (1) angeordnet sind und/oder zusätzlich oder alternativ wenigstens eine Strahlungsquelle (5) für UV-(ultraviolette) Strahlen als Trocknungsvorrichtung (3) und/oder als Keimdestruktionsvorrichtung vorgesehen ist und/oder die Trocknungsvorrichtung (3) derart ausgebildet ist, daß die Trocknungsenergie getaktet abgegeben wird, insbesondere das Mikrowellengerät (4) die Wellen getaktet aussendet und/oder eine Steuerung zumindest für die Taktung des Mikrowellengerätes (4) und/oder der UV-Strahlungsquelle (5) vorgesehen ist.

5. Vorrichtung nach einem oder mehreren der vorhergehenden Ansprüche, **dadurch gekennzeichnet, dass** eine Abluft- bzw. Absaugvorrichtung (6) zum Absaugen der beim Trocknen austretender Wasserdämpfe und Gase vorgesehen ist und/oder in der Trommel (1) eine bevorzugt verschließbare Öffnung (13) zum Füllen und/oder Entleeren der Trommel (1) vorgesehen ist und/oder die Öffnung (13) der Trommel (1) wenigstens an einer Befüllstellung (13a), einer Bearbeitungsstellung (13b) und einer Entleerungsstellung (13c) durch Drehen bzw. Bewegen der Trommel (1) positionierbar ist und/oder die Absaugvorrichtung (6) in der Bearbeitungsstellung (13b) über der Öffnung (13) angeordnet ist.

6. Vorrichtung nach einem oder mehreren der vorhergehenden Ansprüche, **dadurch gekennzeichnet, dass** unter der Vorrichtung korrespondierend zur Entleerungsstellung (13c) ein Auffangbehälter (8) vorgesehen ist und/oder in dem Auffangbehälter (8) ein Transportbehältnis einlegbar ist, welches für einen Weitertransport der aufbereiteten Biomasse geeignet ist und/oder während der Aufbereitung die Zerkleinerungswerkzeuge (2) auf die Biomasse in der Trommel (1) einwirkend ausgebildet sind und/oder wenigstens ein Hammer (21) als Zerkleinerungswerkzeug drehbar um die Achse (11) der Trommel (1) angeordnet ist.

7. Vorrichtung nach einem oder mehreren der vorhergehenden Ansprüche, **dadurch gekennzeichnet, dass** eine vielzahl von Hämmern (21) vorgesehen ist und/oder der oder die Hämmer (21) für ein periodisches Hineinschlagen in das aufzubereitende Gut (7) antreibbar oder ansteuerbar sind und/oder der Hammer (21) an der Welle (11) zu dieser drehbar gelagert ist und ein Mitnehmer (22) vorgesehen ist, der den Hammer (21) bei einer Drehbewegung der Achse (11) mitnimmt und/oder die Drehachse für die Drehbewegung des Hammers (21) außermittig der Achse der Welle (11) vorgesehen ist.

8. Vorrichtung nach einem oder mehreren der vorhergehenden Ansprüche, **dadurch gekennzeichnet, dass** der Mitnehmer (22) auf der Achse (11) beziehungsweise einer Hülse (9) außerhalb des Drehpunktes für den Hammer (21) angeordnet ist und/oder eine Vielzahl von Hämmern (21) auf der Welle (22) vorgesehen ist, wobei die Hämmer (21) beabstandet voneinander und bezüglich ihrer Drehachse versetzt zueinander angeordnet sind und/oder für jeden Hammer (21) ein Mitnehmer (22) vorgesehen ist und/oder die Ansteuerung des Hammers (21) durch die Steuerung der Vorrichtung und/ oder der Trommel (1) realisiert wird und/oder der oder die Hämmer (21) stabförmig ausgebildet sind.

9. Vorrichtung nach einem oder mehreren der vorhergehenden Ansprüche, **dadurch gekennzeichnet, dass** die Hämmer (21) derart aufgehängt sind, daß jeweils das vordere Ende eines Hammerkopfes (24) auf das hintere Ende eines Hammerkopfes (24) eines vorhergehenden Hammers (21) aufschlägt und/oder die Hämmer (21) flügelartig ausgebildet und in Art eines Flügelrades (10) auf einer Hülse (9) drehbar um die Achse (11) der Trommel (1) und in Richtung der Achse (11) relativ zur Trommel (1) verschiebbar angeordnet sind und/oder auf jeder Seite der Trommel (1) wenigstens ein Flügelrad (10) vorgesehen ist und/oder teleskopisch ausgebildete Hülsen (9) für eine Relativbewegung der Hämmer (21) bezüglich der Trommel (1) vorgesehen sind.

10. Vorrichtung nach einem oder mehreren der vorhergehenden Ansprüche, **dadurch gekennzeichnet, daß** als zerkleinerungswerkzeug (2) in der Trommel (1) wenigstens ein relativ zur Antriebsachse (11) rotierbarer, verschiebbarer Hammer (21) vorgesehen ist und/oder die Steuerung zusätzlich oder alternativ die Drehbewegung der Trommel (1), das Befüllen und Entleeren sowie das Absaugen der Wasserdämpfe bzw. Gase regelt und/oder eine Feuchtigkeitsanzeige und/oder Feuchtigkeitsregelung für das aufzubereitende Gut (7) vorgesehen ist und/oder die Feuchtigkeitsregelung derart ausgebildet ist, dass sie die Trocknungsvorrichtung (3) bei vorgegebenen Werten ein- bzw. ausschaltet.

11. Vorrichtung nach einem oder mehreren der vorhergehenden Ansprüche, **dadurch gekennzeichnet, dass** das Gestell (12) rahmenartig ausgebildet ist und/oder die Trommel (1) in dem Gestell (12) drehbar gelagert ist und/oder die Trommel (1) in dem Gestell (12) federgelagert ist.
